# EUROPÄISCHE PATENTANMELDUNG

(11) **EP 0 786 239 A1**
(43) Veröffentlichungstag der Anmeldung: **30.07.1997**
(21) Anmeldenummer: 96890156.1
(22) Anmeldetag: 03.10.1996
(51) Int. Cl.: A61F 5/01

(54) **Schwenkbar miteinander verbundene Knieführungsschienen**

(30) Priorität: 24.01.1996 AT 114/96
(71) Anmelder: GRAFINGER, Josef, A-1160 Wien (AT)
(72) Erfinder: GRAFINGER, Josef, A-1160 Wien (AT)
(74) Vertreter: Rippel, Andreas, Dipl.-Ing.

(57) **Zusammenfassung**

Bei schwenkbar miteinander verbundenen Knieführungsschienen zur Befestigung an Schalen, Bändern und dgl. von Kniegelenkorthesen, Stützapparaten oder an Prothesen, ist ein in einer Oberschiene (2,2') angeordneter Bolzen (3,3') od. dgl. in einer, zwei Führungsflächen aufweisenden, sich nach oben erweiternden Führung (4,4') einer Unterschiene (1,1') geführt. Zur gegenseitigen Abstützung der beiden Schienen (1,2;1',2') ist weiters auf einer der Schienen (1,2;1',2') mindestens eine weitere Führung (8,9;12,13) für die andere der Schienen (1,2;1',2') angeordnet.

Die obere Führungsfläche (5,5') der sich nach oben erweiternden Führung (4,4') ist im wesentlichen eben, die gegenüberliegende Führungsfläche (6,6') gekrümmt.

Eine derartige Anordnung vermag die natürliche Bewegung des Knies, die durch eine kombinierte Dreh- und Gleitbewegung gebildet wird, wirkungsvoll nachzuahmen.

## Beschreibung

Die Erfindung bezieht sich auf schwenkbar miteinander verbundene Knieführungsschienen zur Befestigung an Schalen, Bändern und dgl. von Kniegelenkorthesen, Stützapparaten oder an Prothesen, wobei ein in einer Oberschiene angeordneter Bolzen od. dgl. in einer, zwei Führungsflächen aufweisenden, sich nach oben erweiternden Führung einer Unterschiene geführt ist, und zur gegenseitigen Abstützung der beiden Schienen auf einer der Schienen mindestens eine weitere Führung für die andere der Schienen angeordnet ist

In der frühfunktionellen Behandlung des Kniegelenkes werden Orthesen mit begrenzbarer Gelenkbewegung eingesetzt. Der Einsatz erfolgt dabei z.B. postoperativ in der Kniegelenkschirurgie; zur Immobilisierung nach Knieverletzungen zweiten Grades die keine Operation verlangen und zur späteren Mobilisierung; postoperativ anstelle eines Gipses zur Vermeidung einer Subluxation der Tibia nach anteriorer und posteriorer Kreuzbandverletzung- oder Rekonstruktion; zur sicheren Stabilisierung der Verletzten oder operativ versorgten Seitenbänder.

In vielen Fällen wird der Bewegungsspielraum , sowohl der Extension als auch der Flexion, durch Versetzen von Schrauben auf einer Anschlagplatte begrenzt. Es sind aber auch Orthesen mit freier Gelenkbewegung bekannt.

Um eine größtmögliche Anpassung der gegenseitigen Bewegung der Knieführungsschienen an die natürliche Bewegung des Kniegelenkes zu erreichen, wurden schon die verschiedensten Anordnungen vorgeschlagen und auch verwirklicht. Insbesondere sind Doppelgelenk-Knieführungsschienen in Gebrauch, die als Doppelgelenk-Zahnsegmentschienen ausgeführt sind. Dabei sind beide Schienen mit einem Zahnsegment versehen, welche Segmente miteinander kämmen, und zur Verbindung ist eine gelenkig mit beiden Schienen verbundene Zwischenplatte angeordnet.

Wie jedoch eingehende Untersuchungen gezeigt haben, können auch diese Orthesen den natürlichen Bewegungsablauf des Kniegelenkes nicht nachvollziehen.

Aus der PCT AT 94/00084 sind Knieführungsschienen der eingangs genannten Art bekannt geworden, bei denen in einer ersten Schiene zwei bogenförmige Führungen vorgesehen sind, in die den Schwenkwinkel der beiden Schienen begrenzende Bolzen od. dgl. der zweiten Schiene eingreifen. Dabei greift ein in einer Schiene sitzender weiterer Bolzen od.dgl. in eine weitere bogenförmige Führung der anderen Schiene ein, die sich schräg zur Längsachse dieser anderen Schiene erstreckt. Bei einem bevorzugten Ausführungsbeispiel erweitert sich diese bogenförmige Führung in Richtung von der zweiten Schiene weg.

Eine derartige Anordnung vermag die natürliche Bewegung des Knies, die durch eine kombinierte Dreh- und Gleitbewegung gebildet wird, wirkungsvoll nachzuahmen.

Durch zahlreiche Versuche im Zusammenhang mit theoretischen Überlegungen wurde nun gefunden, daß eine weitere Verbesserung von Knieführungsschienen erreicht werden kann, wenn die obere Führungsfläche (5,5') der sich nach oben erweiternden Führung (4,4') im wesentlichen eben, die gegenüberliegende Führungsfläche (6,6') gekrümmt ist.

Eine solche Führung kann bei Knieführungsschienen der in der PCT-AT-94/00084 beschriebenen Art, aber auch bei anders ausgebildeten Knieführungsschienen angewendet werden. Insbesondere hat sich die Anwendung bei einer Knieführungsschiene der eingangs genannten Art bewährt, bei der, nach einem weiteren Merkmal der Erfindung, die weitere Führung von einer aus zwei gekrümmten, im Mittenbereich einen Höcker aufweisenden, nockenartigen Führungsbahnen gebildet wird, die an der Unterschiene angeordnet sind und an denen sich die bogenförmig ausgebildete Endfläche der Oberschiene abstützt. Eine solche Ausbildung ist besonders einfach und trotzdem äußerst wirkungsvoll.

Vorteilhaft weist dabei die bogenförmig ausgebildete Endfläche der Oberschiene einen Absatz auf, der sich in der etwa gestreckten Stellung der beiden Schienen am Ende der oberen Führungsbahn abstützt. Dadurch wird die Streckstellung der beiden Schienen begrenzt.

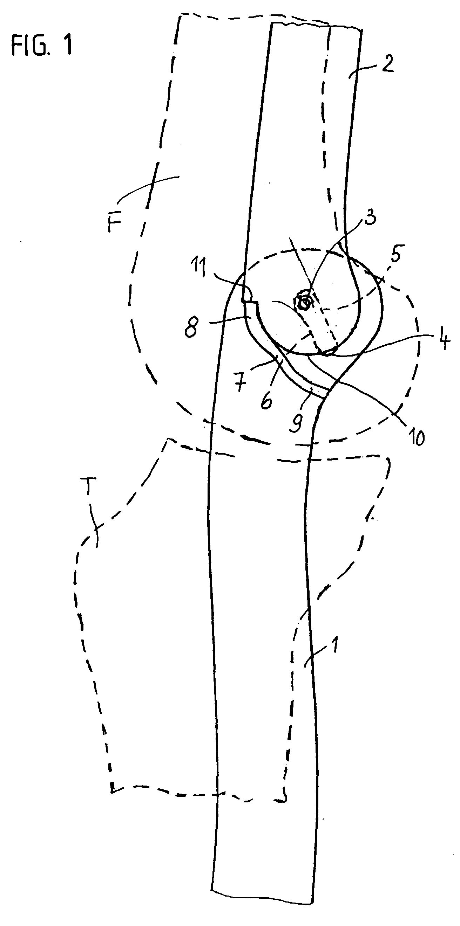

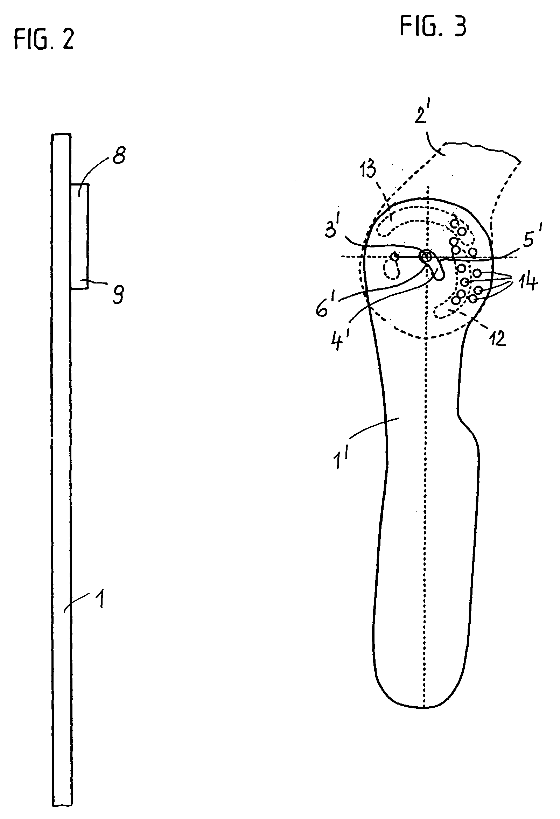

Nachstehend ist die Erfindung anhand von in den Zeichnungen dargestellten Ausführungsbeispielen näher beschrieben, ohne auf diese Beispiele beschränkt zu sein. Dabei zeigen: Fig. 1 schwenkbar miteinander verbundene Knieführungsschienen einer ersten Ausführungsform; Fig. 2 eine Seitenansicht der Unterschiene von Fig. 1; Fig. 3 schwenkbar miteinander verbundene Knieführungsschienen der Art, wie sie in der oben genannten PCT AT 94/00084 beschrieben sind.

Gemäß Fig. 1 sind eine Unterschiene 1 und eine Oberschiene 2 schwenkbar miteinander verbunden. In der Oberschiene 2 ist eine Bohrung angeordnet, in die ein Bolzen 3 od. dgl. eingesetzt ist. Dieser Bolzen oder eine von ihm gehaltene Büchse (nicht dargestellt) greift in eine an der Unterschiene 1 angeordnete Führung 4 ein, die in der aus der PCT AT 94/00084 bekannten Weise schräg nach oben weist.

Erfindungsgemäß ist die obere Führungsfläche 5 der sich nach oben erweiternden Führung 4 im wesentlichen eben, die gegenüberliegende Führungsfläche 6 gekrümmt.

Eine weitere, an der Unterschiene 1 angeordnete Führung wird von einer aus zwei gekrümmten, im Mittenbereich einen Höcker 7 aufweisenden, nockenartigen Führungsbahnen 8 und 9 gebildet, an denen sich die bogenförmig ausgebildete Endfläche 10 der Oberschiene 2 abstützt bzw. bei Verschwenkung der beiden Schienen 1,2 unter teilweisem Gleiten abwälzt.

Diese bogenförmig ausgebildete Endfläche 10 der Oberschiene 2 weist einen Absatz 11 auf, der sich in der in Fig. 1 dargestellten etwa gestreckten Stellung der beiden Schienen 1,2 am Ende der oberen Führungsbahn 8 abstützt. Dadurch wird die Streckstellung der beiden Schienen 1,2 begrenzt.

In Fig. 1 sind etwa die Umrisse von Femur F und Tibia T eingetragen und es ist ersichtlich, daß der durch den Bolzen 3 gebildete Gelenksmittelpunkt annähernd am Epikondylus angesetzt ist.

Die beschriebene und gezeigte Ausführungsform ist vor allem für Stützapparate und auch für Orthesen ohne Bewegungseinschränkung bestimmt und kann auch in hydraulische Kniegelenke, z.B. für Oberschenkelprothesen, eingebaut werden.

Gemäß Fig.3 ist eine Oberschiene 2' und eine Unterschiene 1' vorgesehen. Die Unterschiene 1' weist, wie bei dem eben besprochenen Ausführungsbeispiel, eine bogenförmige Führung 4' auf, die schräg zur Längsachse der Unterschiene 1' und damit zur Längsachse des Unterschenkels verläuft.

Auch bei diesem Ausführungsbeispiel ist die obere Führungsfläche 5' der sich nach oben erweiternden Führung 4' im wesentlichen eben, die gegenüberliegende Führungsfläche 6' gekrümmt.

Die Oberschiene 2' ist mit einer Bohrung versehen, in die eine von einem Bolzen gehaltene Büchse 3' eingesetzt ist, die in der bogenförmigen Führung 4' gleiten kann. Weiters sind in der Oberschiene 2' Führungen 12 und 13 angeordnet, in die in Bohrungen 14 der Unterschiene 2' eingesetzte Bolzen bzw. deren Bolzenköpfe eingreifen können. Diese Bolzen dienen der Begrenzung der Extension und der Begrenzung der Flexion.

## Patentansprüche

1. Schwenkbar miteinander verbundene Knieführungsschienen zur Befestigung an Schalen, Bändern und dgl. von Kniegelenkorthesen, Stützapparaten oder an Prothesen, wobei ein in einer Oberschiene (2,2') angeordneter Bolzen (3,3') od. dgl. in einer, zwei Führungsflächen aufweisenden, sich nach oben erweiternden Führung (4,4') einer Unterschiene (1,1') geführt ist, und zur gegenseitigen Abstützung der beiden Schienen (1,2;1',2') auf einer der Schienen (1,2;1',2') mindestens eine weitere Führung (8,9;12,13) für die andere der Schienen (1,2;1',2') angeordnet ist, **dadurch gekennzeichnet,** daß die obere Führungsfläche (5,5') der sich nach oben erweiternden Führung (4,4') im wesentlichen eben, die gegenüberliegende Führungsfläche (6,6') gekrümmt ist.

2. Schwenkbar miteinander verbundene Knieführungsschienen nach Anspruch 1, **dadurch gekennzeichnet,** daß die weitere Führung (8,9;12,13) von einer aus zwei gekrümmten, im Mittenbereich einen Höcker (7) aufweisenden, nockenartigen Führungsbahnen (8,9) gebildet wird, die an der Unterschiene (1) angeordnet sind und an denen sich die bogenförmig ausgebildete Endfläche (10) der Oberschiene (2) abstützt.

3. Schwenkbar miteinander verbundene Knieführungsschienen nach Anspruch 2, **dadurch gekennzeichnet,** daß die bogenförmig ausgebildete Endfläche (10) der Oberschiene (2) einen Absatz (11) aufweist, der sich in der etwa gestreckten Stellung der beiden Schienen (1,2) am Ende der oberen Führungsbahn (8) abstützt.
